Europäisches Patentamt

European Patent Office

Office européen des brevets

(11) Publication number: **0 069 869**
**B1**

# (12) EUROPEAN PATENT SPECIFICATION

(45) Date of publication of patent specification: **11.09.85**

(21) Application number: **82105219.8**

(22) Date of filing: **15.06.82**

(51) Int. Cl.⁴: **B 01 D 13/04, C 12 N 11/04, A 61 M 1/16, A 61 M 1/18**

(54) A membrane and an arrangement for the removal of a substance from a solution.

(30) Priority: **10.07.81 SE 8104294**
**18.01.82 SE 8200231**

(43) Date of publication of application:
**19.01.83 Bulletin 83/03**

(45) Publication of the grant of the patent:
**11.09.85 Bulletin 85/37**

(84) Designated Contracting States:
**AT BE CH DE FR GB IT LI LU NL**

(56) References cited:
**WO-A-80/02805**
**DE-A-2 060 121**
**DE-A-2 522 821**
**DE-A-2 539 657**
**DE-A-2 650 815**
**DE-A-2 758 507**
**DE-A-2 939 443**
**US-A-3 615 024**
**US-A-4 060 488**
**US-A-4 061 466**
**US-A-4 169 014**
**US-A-4 266 026**

(73) Proprietor: **Gambro Lundia AB**
**Box 10101**
**S-220 10 Lund (SE)**

(72) Inventor: **Sjöholm, Ingvar Gösta Holger**
**Tallmovägen 14**
**S-752 45 Uppsala (SE)**
Inventor: **Edman, Peter Kjell Rudolf**
**Botvidsgatan 16 B**
**S-753 27 Uppsala (SE)**
Inventor: **Nylen, Ulf Thomas Gustav**
**Borgarevägen 15**
**S-222 47 Lund (SE)**

(74) Representative: **Boberg, Nils Gunnar Erik et al**
**Gambro AB Patent Department Box 10101**
**S-220 10 Lund (SE)**

EP 0 069 869 B1

Courier Press, Leamington Spa, England.

## Description

### Technical Field

The present invention relates to a microporous, semipermeable membrane to be used in conjunction with the removal of a substance from a solution, which membrane comprises a biologically active substance being immobilized on and/or within microparticulate gel grains which are located within the pores of said membrane.

The invention relates moreover to an arrangement for the removal of a substance from a solution, this arrangement comprising a microporous, semipermeable membrane which comprises a biologically active substance being immobilized on and/or within microparticulate gel grains which are located within the pores of said membrane.

The expression "removal" in accordance with the invention refers to an actual removal by adsorption or chemical bonding of the substance concerned to the biologically active substance as well as to the removal by chemical or biological conversion of this substance with the help of the biologically active substance.

Examples of applications of actual removal of the substance may be found in connection with therapeutic treatment of a body fluid, especially blood, where undesirable substances, e.g. antibody/antigen complexes are removed by bonding the substance to the biologically active substance, e.g. protein A.

Examples of applications with removal by chemical or biological conversion of the substance may be found in so-called enzyme therapy where an undesirable substance, for example in the blood, is converted by enzymatic action on the substance in contact with the biologically active substance (the enzyme). Other examples may be found in conjunction with purely industrial preparative processes, where a solution containing the substance concerned is brought into contact with the biologically active substance for a conversion of the substance to the desired compound.

Even though the invention consequently has a wide and diverse scope of application, it has been found to be particularly valuable in conjunction with the treatment of whole blood in so-called enzyme therapy and it will be described in the following with the emphasis placed on the last-mentioned application.

### Background Art

Enzyme therapy is a form of treatment where it is endeavoured to replace a lost or diminished enzymatic function in a patient, for example in conjunction with genetically conditioned diseases or as in certain forms of cancer, e.g. lymphoblastic leukaemia, to supply a desired enzymatic function. The treatment in general is taking place so that the enzyme, for example asparaginase in the case of patients with lymphoblastic leukaemia, is allowed to come into contact with the patient's blood in the blood stream for the enzymatic breakdown of a substrate. The enzyme can then break down asparagine, which is essential for the tumour cells, and this means that the tumour cells die owing to lack of nourishment. This can be done by intravenous injection of a solution containing asparaginase into the patient.

The form of treatment described above is unsatisfactory in a number of respects, firstly because as a rule human enzymes are not available, making it necessary to use enzymes from, for example, mammals or bacteria. If such non-human enzymes come into contact with blood corpuscles in the patient's blood antibodies against the enzyme in question will be formed, so that the enzymes become ineffective, since the antibodies will rapidly stop the function of the enzyme. In certain cases anaphylactoid reactions may also occur in the patient as a consequence of the reaction between antibody and enzyme (the antigen).

Even in cases where human enzymes are accessible for the treatment, the problems are considerable, since the stability of enzymes in the patient's bloodstream is often low and it becomes difficult consequently to maintain the enzymatic activity at a satisfactory therapeutic level.

In Chemical Engineering Communications 2 (1975): 1, p. 37—47, a technique is proposed according to which a micoporous, semipermeable, asymmetric membrane is used with a biologically active substance (enzyme) enclosed in the pores of the membrane to make diffusion contact with a solution containing a substance which is to be broken down or in a similar manner removed from the solution.

One disadvantage of this technique consists in that it can only be used in conjunction with high-molecular enzymes, since smaller enzymes may penetrate through the membrane pores and thus obtain free access to the solution which, for example, may be a body fluid.

A further disadvantage can be redistribution of the enzyme (axial enzyme transport) bringing with it impaired stability and consequently lower effectivity.

Similar enzyme systems are also described in US—A—4 169 014, US—A—4 266 026, DE—A—2 939 443 and WO 80/02805.

It is an object of the present invention, therefore, to produce an improved membrane and arrangement for the removal of a substance from a solution in accordance with the technique as described in the abovementioned prior art publications. In particular the invention aims at providing a new form of enzyme therapy by which the aforementioned inconveniences are avoided, regardless of whether human enzymes or enzymes from mammals or bacteria are used.

The abovementioned object is achieved in accordance with the invention with the help of the membrane and the arrangement as defined in the following claims and which will be described in the following text.

Disclosure of Invention

In accordance with the invention is provided a microporous, semipermeable membrane to be used in conjunction with the removal of a substance from a solution, which membrane comprises a biologically active substance being immobilized on and/or within microparticulate gel grains which are located within the pores of said membrane. The membrane is characterized in that said pores are tapering from one side to the other side of the membrane, and in that said microparticulate gel grains with the immobilized biologically active substance are located within said pores at a very short distance from said other side, whereby correspondingly very small diffusion distances are provided.

An asymmetric membrane is known per se from, for example, US patent 3 615 024, and will therefore not be described here in detail. For more detailed information concerning such membranes, reference is made instead to US patent 3 615 024. However, to facilitate understanding of the invention it should be mentioned that asymmetric membranes are made up on the one side by a very thin layer with small pores (barrier layer) and on the other side by a thicker layer with larger pores (support layer), the pores gradually increasing in size from the barrier layer towards the support layer where the pores are thus of maximum size.

In accordance with the invention the asymmetric membrane should have sufficiently large pores in the support layer so that the immobilized biologically active substance, in particular an enzyme, can readily penetrate deeply into the membrane pores from that side, and at the same time have sufficiently small pores on the barrier layer side to prevent the substance from penetrating wholly through the membrane. Furthermore, the membrane in accordance with the invention shall have sufficiently small pores on the barrier layer side to exclude undesirable components when the membrane is intended to be used in conjunction with the treatment of whole blood in enzyme therapy.

At the same time as with the help of the membrane in accordance with the invention contact between the immobilized, enclosed biologically active substance and the solution containing the said substance is avoided, it is an advantage that a very short diffusion distance between the solution containing the said substance and the immobilized active substance can be obtained owing to the immobilized substance being able to penetrate deeply into the pores of the membrane towards the barrier layer side of the membrane which is intended to face towards the solution containing the said substance.

Examples of suitable pore sizes of the membrane in accordance with the invention may be >1 μm, preferably >10 μm on the support layer side, whilst the barrier layer side may have a cutoff of under 150,000 Daltons, preferably 20,000—50,000 Daltons.

The membrane in accordance with the invention may be in the form of a plane sheet or flexible tube, but is preferably in the form of hollow fibres, the solution containing the said substance being intended to pass through the axial cavity of these fibres.

Examples of material used for the membrane in accordance with the invention may be polyamide, cellulose acetate, polysulphone, polycarbonate, polyacrylonitrile, preferably polyamide. With regard to other usable materials, reference is made to the abovementioned US patent 3 615 024.

Suitable microparticulate gel grains may be those described for example in US patent 4 061 466. Preferably these gel grains are spherical particles of a polyacrylic derivative, agarose or polystyrene, preferably polyacrylamide.

In accordance with the invention the said gel grain has a diameter of <10 μm, preferably 0.1—0.5 μm.

Examples of biologically active substances usable may be antibodies, antigens, enzymes, protein A, killed bacteria or fragments thereof, preferably enzymes.

In accordance with the invention an arrangement for the removal of a substance from a solution is also provided, this arrangement comprising a microporous, semipermeable membrane which comprises a biologically active substance being immobilized on and/or within microparticulate gel grains which are located within the pores of said membrane. The arrangement is characterized in that said pores are tapering from one side to the other side of the membrane, and in that said microparticulate gel grains with the immobilized biologically active substance are located within said pores at a very short distance from said other side, whereby correspondingly very small diffusion distances are provided.

The membrane in accordance with the invention described above is intended to be used as the said asymmetric membrane. For more detailed information concerning the same, reference is made therefore to the above description.

In accordance with the preferred embodiment of the arrangement in accordance with the invention, the membrane is used in the form of hollow fibres enclosed within an outer casing with a first inlet and a first outlet communicating with the axial cavity of the fibres. The casing may comprise moreover a second inlet and a second outlet communicating with an outer, closed liquid cycle for a liquid which is intended to be passed through the said casing on the outside of the hollow fibres, as will be described in the following.

The outer liquid cycle preferably comprises a pump for the transport of the said liquid in pulsating flow through the casing on the outside of the hollow fibres. By allowing the pulsating flow to take place with small pulse variations, high and low pressure will be created alternatingly inside the casing, as a result of which the transport of permeating fractions of the solution containing the said substance, flowing through the axial cavity of the hollow fibres, through the hollow fibre walls, to make contact with the enclosed immobilized biologically active substance, is facilitated.

When the arrangement in accordance with the invention is intended to be used for the treatment of whole blood, the outer liquid cycle preferably also comprises a detector for discovering any leakage of whole blood inside the casing.

Brief Description of Drawings

The invention will be described in more detail in the following with reference to the enclosed schematic drawings, wherein

Fig. 1 shows a block diagram of a preferred embodiment of the arrangement in accordance with the invention,

Fig. 2 shows a preferred embodiment of a membrane in accordance with the invention forming part of the arrangement shown in Fig. 1,

Fig. 3 is an enlarged cross-section of the membrane in Fig. 2 taken along line III—III and

Fig. 4 shows a practical embodiment of an outer casing enclosing a membrane in accordance with the invention and intended to be used as part of the arrangement in accordance with the invention shown in Fig. 1.

Best Mode of Carrying Out the Invention

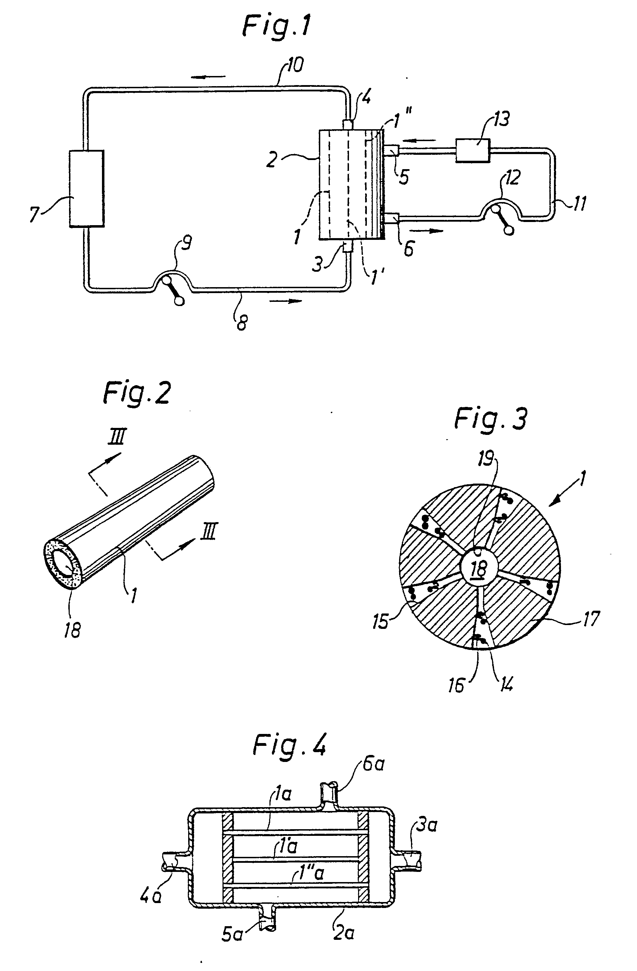

As can be seen schematically in Fig. 1, the arrangement in accordance with the invention comprises an asymmetric membrane (shown schematically by broken lines 1, 1', 1") with enclosing outer casing 2 provided with a first inlet 3 and a first outlet 4 and with a second inlet 5 and a second outlet 6.

The first inlet 3 of the casing 2 is connected to a source 7 for the solution containing the said substance via a conduit 8 comprising a pump 9, whilst the first outlet 4 of the casing 2 is connected to the said source 7 via a return conduit 10 to bring the solution back to this source. The second inlet 5 of the casing 2 is connected to the second outlet 6 via a conduit 11 comprising a pump 12 and a detector 13 forming a closed outer liquid cycle.

As is evident from Fig. 2 and 3, the asymmetric membrane is preferably in the form of hollow fibres 1, preferably of polyamide, with the biologically active substance 14, for example an enzyme, immobilized on a carrier 16 of microparticulate polyacrylamide grains of a size such that they can penetrate easily into the pores 15 through pore openings on the support layer side 17 of the hollow fibres 1 up to a very short distance from the inner wall 19 (barrier layer) of the axial cavity 18. The pore openings on the carrier layer side 19 of the hollow fibres are sufficiently small to prevent these gel grains 16 carrying stabilized biologically active substance 14 from penetrating completely through the pores 15.

In the treatment, for example, of whole blood in conjunction with enzyme therapy these gel grains 16 are of a size of under 10 μm, preferably 0.1—0.5 μm, whilst the pores on the support layer side 17 of the hollow fibres may be greater than 1

μm, preferably greater than 10 μm. The barrier layer 19 of the hollow fibres may have a cut-off of under 150,000 Daltons, preferably 20,000—50,000 Daltons, as a result of which undesirable substances in the whole blood, which is intended to pass through the axial cavity 18 of the hollow fibres, are prevented from penetrating into the pores 15 to make contact with the biologically active substance, for example asparaginase.

It is thus evident from the above that the first inlet 3 communicates with the first outlet 4 via the axial cavity 18 of the hollow fibres 1, 1', 1" inside the casing 2, and that the second inlet 5 communicates with the second outlet 6 via the space which is formed between the casing 2 and the outside of the hollow fibres 1, 1', 1".

In Fig. 4 is shown a practical embodiment of the outer casing shown in Fig. 1 enclosing an asymmetric membrane. The same reference numerals as in Figs. 1—3 have been used therefore in Fig. 4 for corresponding components, but with the addition of the letter "a". As can be seen, the casing 2a is in the form of a conventional fibre dialyzer provided with a first inlet 3a and a first outlet 4a and with a second inlet 5a and a second outlet 6a. The inlet 3a is connected to the outlet 4a via the axial cavity of these fibres, whilst the inlet 5a is connected to the outlet 6a via the space which is formed between the casing 2a and the outside of the enclosed hollow fibres 1a, 1'a, 1"a.

The arrangement functions in the following manner: The solution containing the said substance which is to undergo an enzymatic conversion is conveyed with the help of the pump 9 from its source 7 to the inlet 3 of the casing 2 via the conduit 8 and through the axial cavity of the fibres 1, 1', 1" back to the source 7 via the outlet 4 and the conduit 10. At the same time a liquid, for example a physiological salt solution is pumped into the closed outer liquid cycle with the help of the pump 12 through the casing 2 via the inlet 5 and the outlet 6 on the outside of the hollow fibres 1, 1', 1". During the passage through the axial cavity of the hollow fibres 1, 1', 1" of the solution permeating fractions of the solution containing the said substance will penetrate into the pores 15 to make contact with the biologically active substance 14, preferably an enzyme, to achieve an enzymatic conversion of the said substance. Undesirable substances in the solution, such as antibodies in conjunction with whole blood, are prevented from penetrating into the pores 15, so that a direct contact between these substances and the enclosed biologically active substance 14 is prevented. The penetration of the permeating fractions of the solution is facilitated by the pulsating flow in the outer liquid cycle which is achieved with the help of the pump 12. By means of the detector any rapid leakage of the solution, for example blood inside the casing 2, is rapidly discovered and the system is stopped.

The invention will be illustrated further with the help of the following concrete examples. In all examples a haemofiltration unit of the hollow fibre type (polyamide) from Gambro is used.

### Examples 1—3

In these examples 1—3 the biologically active substance consists of the enzyme asparaginase from E. coli (Crasnitin, Bayer), stabilized on microparticulate polyamide grain (0.2—0.5 µm) enclosed in hollow fibre pores in the manner as described above. The plasma asparagine is determined according to a fluorometric method (D. A. Cooney, R. L. Capizzi and R. E. Handschumacher, Cancer Research 30, 929—935, 1970).

In example 1 a dog (weight 12 kg) is treated with a unit containing 500 U L-asparaginase. The L-asparaginase content in the plasma is reduced from 46 nmole/ml to 2 nmole/ml after 225 minutes treatment. The blood flow was 20 ml/min.

In example 2 the same haemofiltration unit as in example 1 was used with an L-asparaginase solution (2 l) containing 50 nmole L-asparagine per ml. The L-asparagine content is reduced practically speaking completely within 30—40 minutes at a flow of 150—200 ml/min.

In example 3 sheep (weight 40 kg) are treated with a haemofiltration unit of the same type as in example 1 and 2, but containing 2 000 U L-asparaginase in microparticulate polyacrylamide grain. The L-asparaginase content is reduced from normally 40—50 nmole/ml to <10 nmole/ml after 120 minutes treatment at a blood flow of 150—200 ml/min.

A further example to show the application of the present invention in purely industrial processes is the following:

### Example 4

Manufacture of stereospecific amino acids with the help of bacterial enzymes

The stereospecific transformation of ammonium fumarate to L-aspartic acid by means of an amination reaction. The reaction is catalyzed by the enzyme aspartase from E. coli. The enzyme is immobilized in microparticles (0.25—0.5 µm) of the abovementioned type and is installed in the outer space of a haemofiltration unit, for example of the type used in example 1—3 with asymmetric membrane. A solution containing the said substance, which in this example is ammonium fumarate, is pumped through the unit, the substance being brought into contact by diffusion with the aspartase and being converted to L-aspartic acid which is subsequently isolated.

### Example 5

Manufacture of 6-aminopenicillanic acid (6-APA)

The biologically active substance, in this example penicillinacylase from E. coli is immobilized in the asymmetric membrane in a haemofiltration unit as described, whereupon a solution containing raw penicillins is pumped through the unit. The raw penicillins, which are obtained from Penicillum cultures, are converted in the reactor to 6-aminopenicillanic acid, which subsequently is isolated and may be used for specific synthetic penicillins.

### Example 6

Manufacture of glucose and galactose from lactose

In this example β-galactosidase from E-coli or yeast fungus is used as a biologically active substance according to the invention and the procedure is the same as above. A solution containing lactose accordingly will form glucose and galactose by contact with the immobilized biologically active substance inside the haemofiltration unit.

### Industrial Applicability

The arrangement and the membrane in accordance with the invention are applicable for example in conjunction with the enzymatic conversion of a substance present in a solution, as in therapeutic treatments or analyses of body fluids as well as in industrial processes which include an enzymatic conversion.

It is particularly valuable in conjunction with different types of treatments or processes where it is desired to remove a substance from a solution either by adsorption or chemical bonding of the actual substance to a biologically active substance, or by chemical or biological conversion of the substance through contact with the biologically active substance. Examples of the former application may be found in conjunction with a therapeutic treatment of blood where undesirable substances, e.g. antibody/entigen complexes in the blood are removed by means of bonding to the biologically active substance, e.g. protein A. Examples of the latter application may be found in conjunction with so-called enzyme therapy according to which an undesirable substance for example a body fluid, especially whole blood, is removed through enzymatic conversion in contact with the biologically active substance (the enzyme). A further example may be found in conjunction with purely industrial preparative processes, where the substance concerned in contact with the biologically active substance is converted by means of a biological or chemical conversion reaction to a desired compound.

### Claims

1. A microporous semipermeable membrane to be used in conjunction with the removal of a substance from a solution, which membrane comprises a biologically active substance (14) being immobilized on and/or within microparticulate gel grains (16) characterized in that the pores (15) of said membrane are tapering from one side (17) to the other side (19) of the membrane, and in that said microparticulate gel grains (16) with the immobilized biologically active substance (14) are located within said pores (15) at a very short distance from said other side (19), whereby correspondingly very small diffusion distances are provided.

2. A membrane in accordance with claim 1, characterized in that it is present in the form of hollow fibres (1, 1', 1"; 1a, 1'a, 1"a).

3. A membrane in accordance with claim 1 or 2, characterized in that the smallest pore size of the membrane is less than the size of the enclosed microparticulate gel grains (16) carrying immobilized biologically active substance (14).

4. A membrane in accordance with any of claims 1—3, characterized in that the pore size on said one side (17) of the membrane is >1 μm, preferably >10 μm.

5. A 'membrane in accordance with any of claims 1—4, characterized in that the membrane has a cut-off of 150,000 Daltons, preferably 20,000—50,000 Daltons.

6. A membrane in accordance with any of claims 1—5, characterized in that said gel grains (16) have a diameter of <10 μm, preferably 0.1—0.5 μm.

7. A membrane in accordance with any one of claims 1—6, characterized in that the said gel grains (16) are selected among polyacrylamide, agarose and polystyrene, preferably polyacrylamide.

8. A membrane in accordance with any one of claims 1—7, characterized in that it is selected among polyamide, cellulose acetate, polysulphone, polycarbonate, polyacrylonitrile, preferably polyamide.

9. A membrane in accordance with any one of the preceding claims, characterized in that the biologically active substance is selected among antibodies, antigens, protein A, killed bacteria or fragments thereof and enzymes such as asparaginase, aspartase from E. coli, penicillanic acid from E. coli, β-galactosidase from E. coli and yeast fungus.

10. An arrangement for the removal of a substance from a solution, this arrangement comprising a microporous, semipermeable membrane which comprises a biologically active substance (14) being immobilized on and/or within microparticulate gel grains (16) characterized in that the pores (15) of said membrane are tapering from one side (17) to the other side (19) of the membrane, and in that said microparticulate gel grains (16) with the immobilized biologically active substance (14) are located within said pores (15) at a very short distance from said other side (19), whereby correspondingly very small diffusion distances are provided.

11. An arrangement in accordance with claim 10, characterized in that the said membrane is present in the form of hollow fibres (1, 1', 1"; 1a, 1'a, 1"a).

12. An arrangement in accordance with claim 11, characterized in that the said hollow fibres are enclosed within an outer casing (2; 2a) with a first inlet (3; 3a) and a first outlet (4; 4a) communicating with the axial cavity (18) of the fibres.

13. An arrangement in accordance with claim 12, characterized in that the said casing (2; 2a) comprises a second inlet (5; 5a) and a second outlet (6; 6a) respectively communicating with an outer liquid cycle for a liquid which is intended to pass through the said casing on the outside of the hollow fibres.

14. An arrangement in accordance with claim 13, characterized in that the said outer liquid cycle comprises a pump (12) preferably a so-called roller pump, for circulation of the said liquid in this cycle.

15. An arrangement in accordance with claim 13 or 14, characterized in that the said outer cycle comprises a detector (13) for the detecting of possible leakage of solution containing the said substance within the casing (2; 2a).

## Revendications

1. Membrane semi-perméable microporeuse utilisable en conjonction avec l'élimination d'une substance d'une solution, cette membrane comprenant une substance biologiquement active (14) immobilisée sur et/ou dans des grains de gel (16) en micoparticules, caractérisée en ce que les pores (15) de la membrane ont une configuration convergente, d'un premier côté (17) à l'autre côté (19) de la membrane, et en ce que ces grains de gel (16) en microparticules portant la substance biologiquement active (14) immobilisée sont situés dans les pores (15) à une très faible distance du dit autre côté (19), de sorte qu'on obtient de très petites distances de diffusion, de façon correspondante.

2. Membrane suivant la revendication 1, caractérisée en ce qu'elle est sous la forme de fibres creuses (1, 1', 1"; 1a, 1'a, 1"a).

3. Membrane suivant la revendication 1 ou 2, caractérisée en ce que la plus petite dimension de pore de la membrane est inférieure à la dimension des grains de gel (16) en micoparticules contenus dans les pores et portant la substance biologiquement active (14) immobilisée.

4. Membrane suivant l'une quelconque des revendications 1 à 3, caractérisée en ce que la dimension de pore du dit premier côté (17) de la membrane est supérieure à 1 μm et de préférence supérieure à 10 μm.

5. Membrane suivant l'une quelconque des revendications 1 à 4, caractérisée en ce que la membrane a une coupure de 150 000 Daltons et de préférence de 20 000 à 50 000 Daltons.

6. Membrane suivant l'une quelconque des revendications 1 à 5, caractérisée en ce que les grains de gel (16) ont un diamètre inférieur à 10 μm, et de préférence de 0,1 à 0,5 μm.

7. Membrane suivant l'une quelconque des revendications 1 à 6, caractérisée en ce que les grains de gel (16) sont choisis parmi des substances telles que polyacrylamide, agarose et polystyrène, et de préférence polyacrylamide.

8. Membrane suivant l'une quelconque des revendications 1 à 7, caractérisée en ce qu'elle est choisie parmi les matières telles que polyamide, acétate de cellulose, polysulfone, polycarbonate, polyacrylonitrile, et de préférence polyamide.

9. Membrane suivant l'une quelconque des revendications précédentes, caractérisée en ce que la substance biologiquement active est choisie parmi des substances telles qu'anticorps, antigènes, protéines A, bactéries tuées ou leurs fragments et enzymes, par exemple asparaginase, aspartase de E. coli, acide penicillanique de E. coli, β-galactosidase de E. coli et champignon de levure.

10. Dispositif pour éliminer une substance d'une solution, ce dispositif comprenant une membrane semi-perméable microporeuse qui contient une substance biologiquement active (14) immobilisée sur et/ou dans des grains de gel (16) en microparticules, caractérisé en ce que les pores (15) de la membrane ont une configuration convergente, d'un premier côté (17) à l'autre côté (19) de la membrane, et en ce que ces grains de gel (16) en microparticules portant la substance biologiquement active (14) immobilisée sont situés dans les pores (15) à une très faible distance du dit autre côté (19), de sorte qu'on obtient en correspondance de très petites distances de diffusion.

11. Dispositif suivant la revendication 10, caractérisé en ce que la membrane se présente sous la forme de fibres creuses (1, 1', 1"; 1a, 1'a, 1"a).

12. Dispositif suivant la revendication 11, caractérisé en ce que les fibres creuses sont contenues dans une enveloppe extérieure (2; 2a) comportant une première entrée (3; 3a) et une première sortie (4; 4a) qui communiquent avec la cavité axiale (18) des fibres.

13. Dispositif suivant la revendication 12, caractérisé en ce que l'enveloppe (2; 2a) comporte une deuxième entrée (5; 5a) et une deuxième sortie (6; 6a) qui communiquent respectivement avec un circuit de liquide extérieur pour un liquide destiné à passer dans l'enveloppe du côté extérieur des fibres creuses.

14. Dispositif suivant la revendication 13, caractérisé en ce que le circuit de liquide extérieur comprend une pompe (12), de préférence une pompe dite à galets, pour faire circuler le dit liquide dans ce circuit.

15. Dispositif suivant la revendication 13 ou 14, caractérisé en ce que le circuit extérieur comprend un détecteur (13), pour la détection d'une fuite éventuelle de solution contenant la dite substance à l'intérieur de l'enveloppe (2; 2a).

**Patentansprüche**

1. Mikroporöse semipermeable Membran für die Verwendung in Verbindung mit der Entfernung einer Substanz aus einer Lösung, mit einer biologisch aktiven Substanz (14), die auf und/oder in mikrofeinteiligen Gelkörnern (16) immobilisiert ist, dadurch gekennzeichnet, daß die Poren (15) der Membran von einer Seite (17) zu der anderen Seite (19) der Membran sich verjüngen und daß die mikrofeinteiligen Gelkörner (17) mit der immobilisierten biologisch aktiven Substanz (14) in diesen Poren (15) in einem sehr kurzen Abstand von der anderen Seite (19) angeordnet sind, wodurch entsprechend sehr kleine Diffusionsabstände gebildet werden.

2. Membran nach Anspruch 1, dadurch gekennzeichnet, daß sie in der Form von Hohlfasern (1, 1', 1"; 1a, 1'a, 1"a) vorliegt.

3. Membran nach Anspruch 1 oder 2, dadurch gekennzeichnet, daß die kleinste Porengröße der Membran kleiner als die Größe der eingeschlossenen mikrofeinteiligen Gelkörner (16), die die immobilisierte biologisch aktive Substanz (14) tragen, ist.

4. Membran nach einem der Ansprüche 1 bis 3, dadurch gekennzeichnet, daß die Porengröße auf der einen Seite (17) der Membran >1 μm, vorzugsweise >10 μm ist.

5. Membran nach einem der Ansprüche 1 bis 4, dadurch gekennzeichnet, daß die Membran eine Ausschlußgrenze von 150 000 Dalton, vorzugsweise 20 000 bis 50 000 Dalton, besitzt.

6. Membran nach einem der Ansprüche 1 bis 5, dadurch gekennzeichnet, daß die Gelkörner (16) einen Durchmeser von <10 μm, vorzugsweise von 0,1 bis 0,5 μm haben.

7. Membran nach einem der Ansprüche 1 bis 6, dadurch gekennzeichnet, daß die Gelkörner (16) unter Polyacrylamid, Agarose und Polystyrol ausgewählt sind und vorzugsweise aus Polyacrylamid bestehen.

8. Membran nach einem der Ansprüche 1 bis 7, dadurch gekennzeichnet, daß sie unter Polyamid, Celluloseacetat, Polysulfon, Polycarbonat und Polyacrylnitril ausgewählt ist und vorzugsweise aus Polyamid besteht.

9. Membran nach einem der vorausgehenden Ansprüche, dadurch gekennzeichnet, daß die biologisch aktive Substanz unter Antikörpern, Antigenen, Protein A, abgetöteten Bakterien oder Bruchstücken derselben und enzymen, wie Asparaginase, Aspartase aus E. coli, Penicillansäure aus E. coli, β-Galactosidase aus E. coli und Hefepilzen, ausgewählt ist.

10. Vorrichtung zur Entfernung einer Substanz aus einer Lösung mit einer mikroporösen semipermeablen Membran, die aus einer biologisch aktiven Substanz (14) besteht, die auf und/oder in mikrofeinteiligen Gelkörnern (16) immobilisiert ist, dadurch gekennzeichnet, daß die Poren (15) der Membran von einer Seite (17) zu der anderen Seite (19) der Membran sich verjüngen und daß die mikrofeinteiligen Gelkörner (16) mit der immobilisierten biologisch aktiven Substanz (14) in diesen Poren (15) in einem sehr kurzen Abstand von der anderen Seite (19) angeordnet sind, wodurch entsprechend sehr kleine Diffusionsabstände gebildet werden.

11. Vorrichtung nach Anspruch 10, dadurch gekennzeichnet, daß die Membran in der Form von Hohlfasern (1, 1', 1"; 1a, 1'a, 1"a) vorliegt.

12. Vorrichtung nach Anspruch 11, dadurch gekennzeichnet, daß die Hohlfasern in einem Außengehäuse (2; 2a) mit einem ersten Einlaß (3; 3a) und einem ersten Auslaß (4; 4a), die in Verbindung mit dem axialen Hohlraum (18) der Hohlfasern stehen, eingeschlossen sind.

13. Vorrichtung nach Anspruch 12, dadurch gekennzeichnet, daß das Gehäuse (2; 2a) einen zweiten Einlaß (5; 5a) bzw. einen zweiten Auslaß (6; 6a), die mit einem äußeren Flüssigkeits-

**0 069 869**

kreislauf für eine Flüssigkeit, die durch das Gehäuse auf der Außenseite der Hohlfasern gehen soll, verbunden sind, aufweist.

14. Vorrichtung nach Anspruch 13, dadurch gekennzeichnet, daß der äußere Flüssigkeitskrieslauf eine Pumpe (12), vorzugsweise eine sogenannte Rollenpumpe, für eine Zirkulation der Flüssigkeit in dem Kreislauf aufweist.

15. Vorrichtung nach Anspruch 13 oder 14, dadurch gekennzeichnet, daß der äußere Kreislauf einen Detektor (13) für die Feststellung einer möglichen Leckage von die Substanz enthaltender Lösung in dem Gehäuse (2; 2a) aufweist.

0 069 869

*Fig.1*

*Fig.2*

*Fig. 3*

*Fig. 4*

1